Europäisches Patentamt

European Patent Office

Office européen des brevets

(19)

(11) Publication number: **0 109 205**
**A2**

(12) **EUROPEAN PATENT APPLICATION**

(21) Application number: **83306359.7**

(22) Date of filing: **19.10.83**

(51) Int. Cl.³: **A 61 B 6/02**

(30) Priority: **15.11.82 US 441857**

(43) Date of publication of application: **23.05.84**
**Bulletin 84/21**

(84) Designated Contracting States: **DE FR GB NL**

(71) Applicant: **PICKER INTERNATIONAL, INC., 595 Miner Road, Highland Heights Ohio 44143 (US)**

(72) Inventor: **DeMeester, Gordon D., 7701 Sharon Drive, Mentor Ohio 44060 (US)**
Inventor: **Brunnett, Carl J., 1650 Mayfair Boulevard, Mayfield Heights Ohio 44124 (US)**
Inventor: **Kenue, Surender K., 10301 Lake Avenue, 716 Cleveland Ohio 44124 (US)**

(74) Representative: **Pope, Michael Bertram Wingate, Central Patent Department The General Electric Company, p.l.c. Hirst Research Centre East Lane, Wembley Middlesex HA9 7PP (GB)**

(54) **Method and apparatus for computed tomography imaging.**

(57) Method and apparatus for approximating x-ray detector data from a malfunctioning computed tomography detector. A view of data is gathered for each of a plurality of detectors in a stationary detector scanner. Data from malfunctioning detectors is approximated by averaging the data from closely adjacent detectors after a preferred gradient is chosen to establish which detector data is to be averaged.

EP 0 109 205 A2

9-364

## Description

## Method and Apparatus for
## Computed Tomography Imaging

### Technical Field

The present invention relates to computed tomography and more particularly to a method and apparatus for approximating detector data for mal-functioning x-ray detector channels in such a scanner. Ⓐ

### Background Art

The last decade has seen the practice of computed tomography mature from an experimental technique used Ⓐ to an accepted diagnostic procedure used in hospitals throughout the world. The computed tomography diagnosing technique differs from conventional x-ray radiation Ⓐ technique in that an image representing density variations across a patient slice can be obtained using the computed tomography technique whereas conventional x-rays yield only a shadow image of the patient cross-section. A physician viewing a conventional x-ray must be truly skilled to determined differences in density between structures on the x-ray. The physician viewing an output from a computed tomography apparatus, however, can immediately discern differences in density from the image presented to him.

In computed tomography a patient or subject cross-section of interest is successively scanned from a multitude of orientations by an x-radiation source which directs x-rays through the patient slice of interest. One or more detectors positioned on an opposite side of the patient from the source obtain intensity readings of the x-radiation after it is passed through the patient. If enough intensity measurements or readings from a variety of orientations are obtained, these intensity readings can be utilized according to one of a number of reconstruction algorithms to yield a density mapping of the patient cross-section. The reconstruction technique is derived from mathematical reconstruction algorithms dating back to the beginning of the twentieth century. Each intensity value of radiation passing through the patient corresponds to a line integral of an attenuation function taken through the patient cross-section from the source to the position the intensity is sensed.

A first generation of computed tomography devices were quite slow, and produced low resolution images of the objects being scanned. This first generation of machines, however, did validate the basic computed tomography techniques and suggested that with refinement the computed tomography procedure could be utilized to aid the physician in diagnosing internal structure of a patient.

The first generation machines comprised a single source of x-radiation and a single detector which were caused to scan in a linear fashion past a subject a number of times at a number of different orientations. In operation, the first generation machines used a linear scan technique whereby detector and source were repetitively translated, rotated and retranslated to produce intensity readings suitable for analysis in reconstruction. These first generation machines were slow. They could take on the order of five minutes to obtain enough intensity readings to enable state of the art computers to generate a crude image. The slowness of the process and lack of resolution in the final image were serious concerns that had to be overcome before computed tomography could be routinely utilized as a tool for useful diagnosis.

A second generation of computed tomography devices operated on the same principle that the first generation developed but significantly increased resolution while speeding the time it took to provide a useable image. The second generation device included a source of x-radiation and a bank or array of detectors which moved in unison with the source in a motion similar to the first generation machine i.e. translations and rotations were successively alternated to irradiate the patient from a number of orientations thereby producing enough intensity readings to reconstruct an image. It was the second generation of computed tomography apparatus which became commercially viable and was sold for use by hospitals in diagnosing the internal structure of patients.

A third generation of computed tomography apparatus resulted in even faster imaging. Third generation machines no longer required alternating translations and rotations to produce an image. The motion in the third generation machines is characterized as orbital wherein a single source and a bank or array of detectors orbits in unison about a patient. In a third generation CT machine time was no longer wasted in stopping and starting the mechanical arrangement to which source and detectors are mounted. The third generation computed tomography machines were much faster but suffered from deficiencies in data sampling and a so-called ring artifact. The deficiencies have been documented in the literature and need not be discussed at length except to point out that the enhancements and speed provided by the third generation device were substantially offset by them. THESE DEFICIENCIES

Third generation computed tomography scanning systems are still being marketed as are CT devices built in accordance with a fourth generation design. The fourth generation design includes an array of detectors which are stationary with respect to a moving x-ray source. Typically the fourth generation array of detectors completely surround a patient aperture through which the patient is positioned for CT scanning. An x-ray source then irradiates the patient from a number of different orientations which is most typically achieved by orbiting or rotating the x-ray source about the patient. The fourth generation design eliminates the stopping and starting action common to the first and second generation systems so the speed advantages of the orbital type arrangement (third generation) are achieved and often exceeded and the ring artifact problem is not experienced. The time period required to produce a patient image in a typical fourth generation CT scanning system has been reduced to the order of a second and it is perceived that this time can be cut even further as computed tomography techniques are enhanced.

One feature common to all computed tomography systems is that they must include apparatus for detecting x-radiation intensity that has passed through the patient. In the first generation CT systems this apparatus involved a single detector which moved in unison with the moving source. The second and third generation CT systems included a plurality of detectors also moving with respect to the patient and in the fourth generation systems a high number of detectors are used.

Early computed tomography used one of two types of x-radiation detectors. One type detector is the scintillation detector which used a crystal for converting x-radiation impinging upon it into visible light which was then detected by a photomultiplier tube. This tube converted the light into an electrical current and amplified this current to a level suitable for measuring the intensity.

Gas ionization chambers are also used in some CT designs. In such a chamber the high energy radiation passing from the source through the patient to the detector causes an ionization of the gas stored in the ionization chamber.  By measuring the current generated in this ionization process, an indication of x-radiation intensity is obtained.

Photodiodes have increasingly replaced the photomultiplier tube techniques for detecting light from a scintillation crystal utilized in a CT environment.  These detectors typically comprises a scintillation crystal mounted in close proximity to a photodiode which produces an output current proportional to the x-ray intensity striking the scintillation crystal.

In a fourth generation environment, for example, an array of 600 or more closely packed photodiodes are positioned in a circular arrangement to circumscribe a patient aperture so that x-radiation from a rotating x-ray tube impinges upon each of the detectors comprising the array as the x-ray tube orbits around the patient.  Electronics coupled to each of the detectors convert the current output from each photodiode into a digital signal proportional to the x-ray intensity impinging upon a given detector.

The detectors have advantages over gas ionization or photomultiplier tube detectors.  Perhaps the most significant advantage the solid state detectors have is the very close packing density these detectors permit in a fourth generation machine.  A typical scintillation crystal and photodiode is mounted in a package sufficiently small to allow well over one thousand detectors to be positioned in a reasonable space for scanning.

In a CT apparatus requiring an excess of one thousand detectors mounted in a circular array about a subject aperture, it is possible that certain detectors may malfunction and no longer produce meaningful output data.  It is also possible that the elctronic circuitry

6    0109205

for analysing the output from the detectors will also, on occassion, malfunction to the point where the output from the circuitry may be inaccurate or unreliable. In either event, it is necessary that the bad detector or circuit (detector channel) be ignored for reconstruction purposes.

In the past when a detector or circuit has gone bad, an intensity reading has been assigned to the bad detector by averaging the intensity outputs from the two detectors closely adjacent to the bad detector. Experience with this simple averaging technique indicates that it is not sufficient since using a simple averaging leads to artifacts in the reconstructed picture. These artifacts typically comprise streaks across the reconstructed image which correspond to no internal structure in the subject. These streakings or artifacts occur because incorrect values are assigned to the sample in the averaging technique. It is apparent therefore, that a more sophistocated technique for supplying intensity readings to bad detectors and/or circuits is required if these artifacts are to be avoided.

Disclosure of the Invention

Computed tomography images constructed in accordance with the present invention avoid the artifacts associated with prior art bad detector approximations by making those approximations more accurate. Stated another way, if one could compare the approximations used in accordance with the present invention with the output data from a detector, were it working, the comparison would be more accurate than the simple averaging of those detectors next to the malfunctioning detector. The simple averaging of detector outputs is replaced by a more selective choice of data for use in approximating the output from a nonfunctioning or "bad detector."

To best appreciate the invention, it is constructive to recall how computed tomography intensity read-

7        0109205

ings are obtained.  As a moving x-radiation source follows a generally circular path about a subject of interest, closely spaced detectors measure the intensity of
the x-radiation as it is attenuated by the subject.
These intensity readings are taken at successive time
intervals so that as a given detector is irradiated, a
number of successive intensity readings from that de-
tector will be sensed, converted to a digital output,
and stored in a computer memory.  One can plot the output of detectors during a computed tomography scan on
an orthogonal grid system where the detectors are
listed along the ordinate scale and the spatial
position of the measurement is the abscissa.  The
measurements from one detector is known as a view.

     In accordance with the present invention various
differences are taken between the output from closely
spaced functioning detectors.  These differences are
compared and a minimum difference chosen as a preferred
difference.  This preferred difference is then used to
define a gradient or direction of the grid of detector
verses position mapping.  This is to be contrasted with
the prior art technique where only one direction could
be chosen on this mapping, i.e. a vertical direction
corresponding to the direction defined by two adjacent
functioning detectors at the same relative position of
each detector's view.

     The choice of a minimum difference between adjacent
detectors reflects a decision that the detector data to
be preferred when assigning values for defective detectors is that data which represent a smooth or gradual
change in value rather than sharp, abrupt discontinuities.  The mere averaging of detector outputs sometimes
produces these discontinuities and it is seen through
practice of the invention that using a minimum gradient
approach reduces the tendancy to have such abrupt changes.

Once a preferred direction is defined on the detector/position mapping, the data falling along this direction is used to approximate a value for the bad detector which is no longer producing a valid output reading. In its simplest embodiment, the two closest readings to the faulty detector location are averaged together and assigned to the missing detector. In more complex embodiments of the invention, a weighted average of ScALED̶ outputs closely spaced about the missing detector location is assigned to that location.       (A)

The preferred technique for approximating intensity outputs is not limited to a single detector. If two adjacent detectors are not functioning, the detectors surrounding those malfunctioning detectors can be used to approximate data for both detectors. The technique used in approximating data for two adjacent detectors is similar to the single detector instance but requires more intensity readings on either side of the malfunctioning pair.

In a computed tomography scanner having 1200 detectors, the disclosed invention allows meaningful computed tomography images to be created even though many detectors have malfunctioned. If a large number of detectors have malfunctioned it is preferable that they be spaced throughout the array in single or small groups so that sufficient data from surrounding detectors allows the data missing from the malfunctioning detectors to be approximated accurately.

From the above it should be appreciated that one object of the present invention is the creation of computed tomography images from a stationary detector computed tomography scanner wherein certain ones of the detectors comprising the stationary detector array are malfunctioning. In particular, the artifacts associated with the prior art bad detector approximations are avoided

9                    0109205

through an approximation technique which. analyses the
reasonableness of the data from adjacent detectors and
optimizes these readings to produce a preferred gradient.
These and other objects, advantages and features of the
invention will be described in greater detail in conjunction with the accompanying drawings.

Brief Description of the Drawings

Figure 1 is a perspective view of a computed tomography (CT) scanner of the stationary detector design
and the peripheral components of that scanner;

Figure 2 is a front elevational view of the CT
scanner schematically depicting the major components
including an air conditioning system;

Figure 3 is a perspective view of a detector module in relation to a stationary scanner gantry;

Figure 4 is a schematic of a series of steps performed to reconstruct a computed tomography image;

Figure 5 shows a fan beam of radiation directed
across a cross-section subject;

Figure 6 is a plot of the data stored in a computer
corresponding to the outputs from a CT detector as it
senses radiation passing through the subject of Figure
5; and

Figure 7 is digital filtered data corresponding to

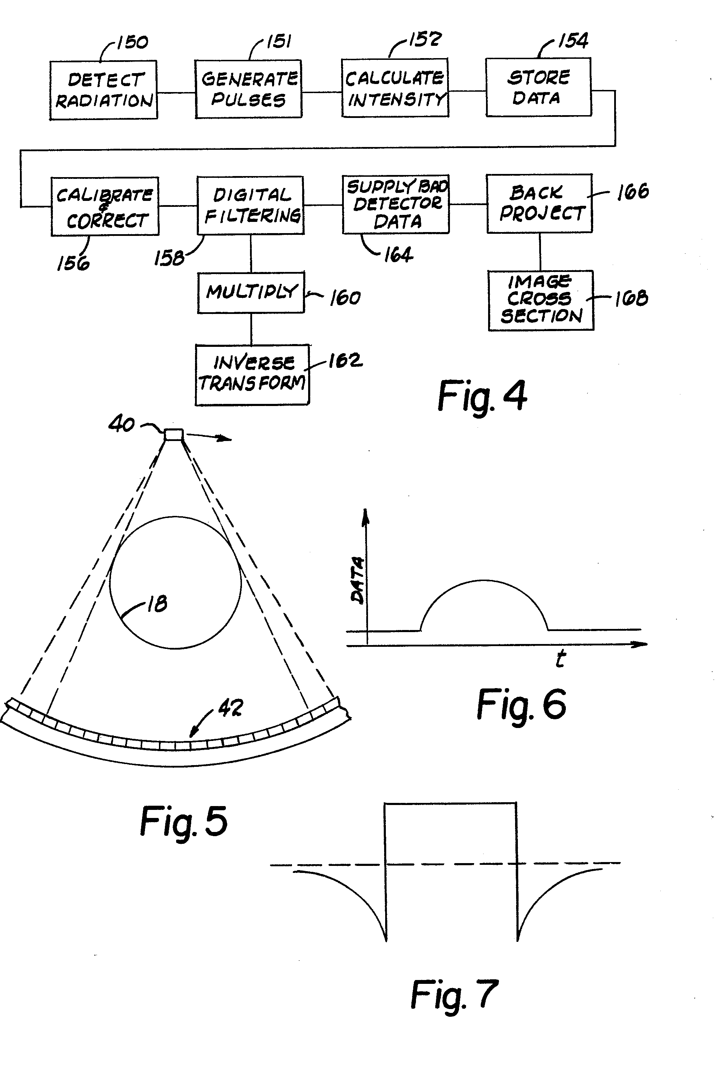

the Figure 6 data.

Best Mode for Carrying out the Invention

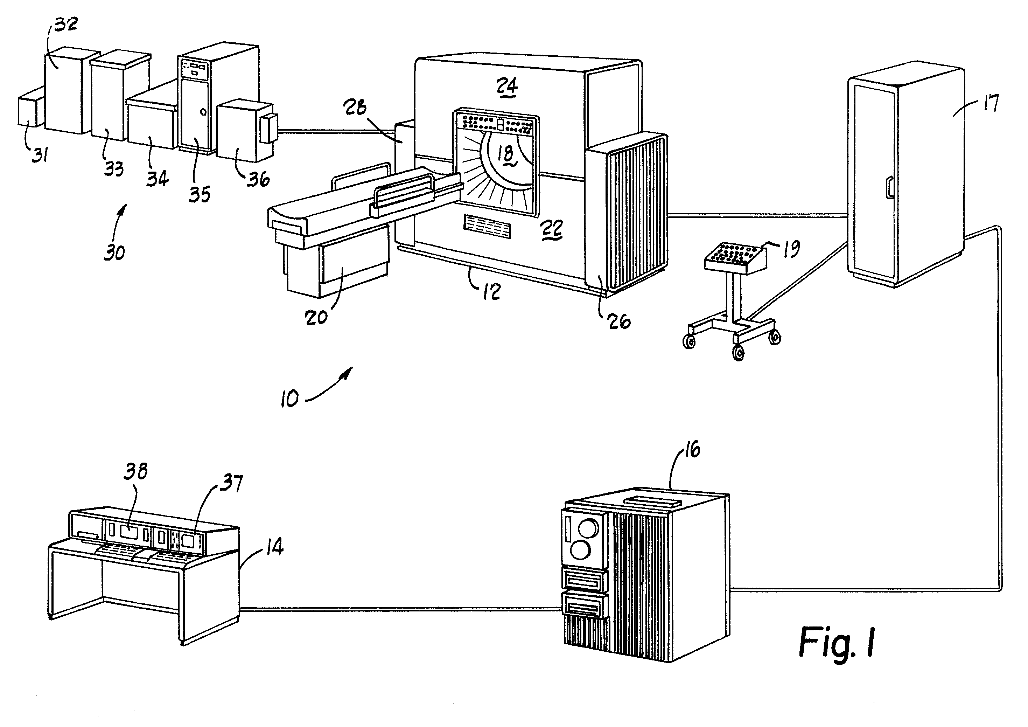

Turning now to the drawings, Figure 1 illustrates
a computed tomography scanning system 10 used in imaging
cross-sectional slices of interest in a patient of inte-
rest. The computed tomography system 10 comprises a
scanner 12, a viewing console 14, a computer 16, and
specialized electronics 17 needed by the scanner 12 for
control and data handling.

The scanner 12 is a fourth generation computed
tomography scanner where a fixed array of detectors surrounds a patient aperture 18. During imaging a patient is positioned on a couch 20 and then moved into and through the patient aperture 18 until a cross-sectional slice to be imaged is appropriately positioned. A scanner front panel is divided into two portions 22, 24 which are hinged to the scanner housing. These two portions swing away from the position they are shown in Figure 1 to allow the interior of the scanner 12 to be accessed. The scanner housing is supported by a pair of supports 26, 28 and can be tilted about an axis extending through the supports parallel to the floor. In this way, patient cross-sections other than a vertical cross-section can be obtained without repositioning the patient.

The generation of x-radiation requires a series of electronic subsystems 30 shown to the side of the computed tomography scanner 12. In an x-ray tube, highly accellerated electrons are directed to a tube anode from a cathode and in particular electrons having nearly 150,000 electron volts of energy strike the anode to produce x-radiation. To accellerate the electrons, a voltage of nearly 150,000 volts must be transmitted from the electronics 30 to rotating portions of the scanner 12. The electronics needed to generate these high voltages includes a heat exchanger 31, a power module 32, a high voltage transformer 33, a voltage regulator 34, an x-ray control module 35, and a high speed starter 36.

In computed tomography scanning, special electronics needed to analyze intensity values as detected by the scanner 12 are also required. This specialized electronics 17 counts output pulses from the scanner detectors as well as controls movement of an x-ray tube and coordinates this movement with the analysis of the output signals. A service module 19 coupled to the specialized electronics 17 allows the scanner 12 to be tested without the aid of the computer 16 or the viewing console 14.

High speed computed tomography scanning is possible only through use of a high speed data processing computer 16. The illustrated and presently preferred computer 16 is a 32 bit Perkin-Elmer mini computer with a disc storage capacity of 320 million bytes. This computer 16 performs the sophisticated data processing for reconstructing a grid-like mapping of density variations inside the patient slice from intensity readings taken from a plurality of detectors surrounding the patient aperture. The particular computer chosen is responsible for not only analysing and reconstructing cross-sectional image densities but also for displaying this information on a console 14.

The console depicted in Figure 1 includes a first work station 37 for a technician operating the computed tomography apparatus and a second work station 38 for a person responsible for diagnosing the images produced. Although not shown in Figure 1, a remote diagnosing station is optionally provided so that the person diagnosing the patient need not be in the same location as the operator.

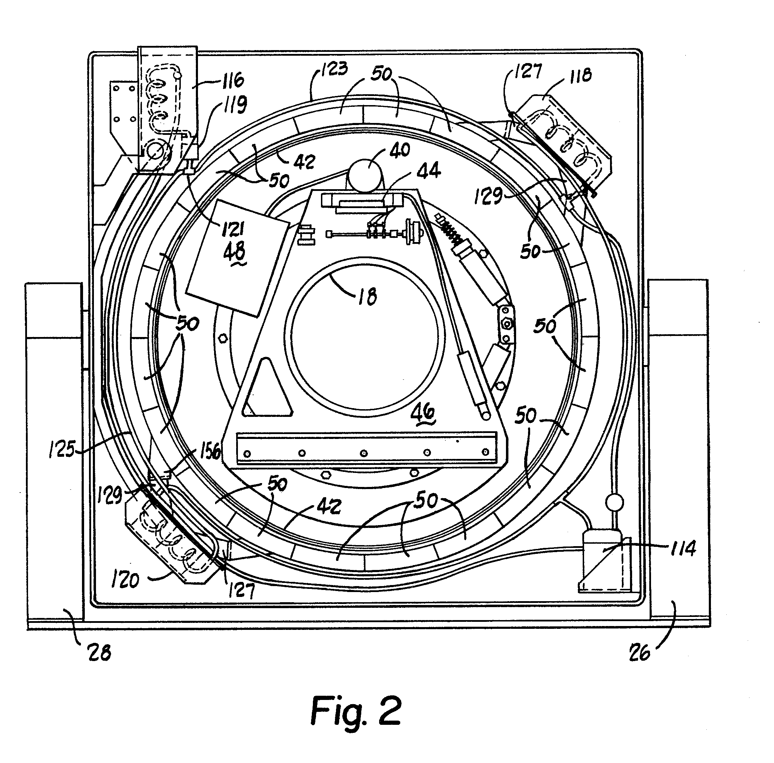

When the panel portions 22, 24 are pivoted away from the position shown in Figure 1, an x-ray tube 40 and fixed array 42 of x-radiation detectors are visible (see Figure 2). The illustrated array 42 completely encircles the patient aperture 18. During scanning, the x-ray tube 40 generates an x-ray beam which is shaped by a collimator 44 to provide a generally planar spread beam of radiation that passes through the aperture 18 to opposed detectors in the array 42. The x-radiation is attenuated by the patient (or any other objects in the path of the x-radiation) and its intensity is then detected by detectors in the array 42. The tube 40 and collimator 44 are both mounted to a CT frame 46 which is rotatably mounted to the scanner 12. A motor (not shown) at the rear of the CT apparatus imparts rotational motion to the frame 46 which is journalled on stationary portions of the scanner 12 by a bearing (not shown) which surrounds the patient aperture.

The x-ray tube 40 heats during operation and in particular, unless it is cooled, the tube 40 can overheat and malfunction. For this reason, a cooling unit 48 is mounted to the side of the x-ray tube and coupled to the tube to circulate coolant to the tube housing.

The array 42 of detectors is made up of individual modules 50 which each support a plurality of closely spaced detectors in fixed relation around the patient aperture 180. In accordance with a preferred design, each module 50 supports sixty detectors 51 so that the 20 modules comprising the array 42 supports 1200 detectors in a circular array.

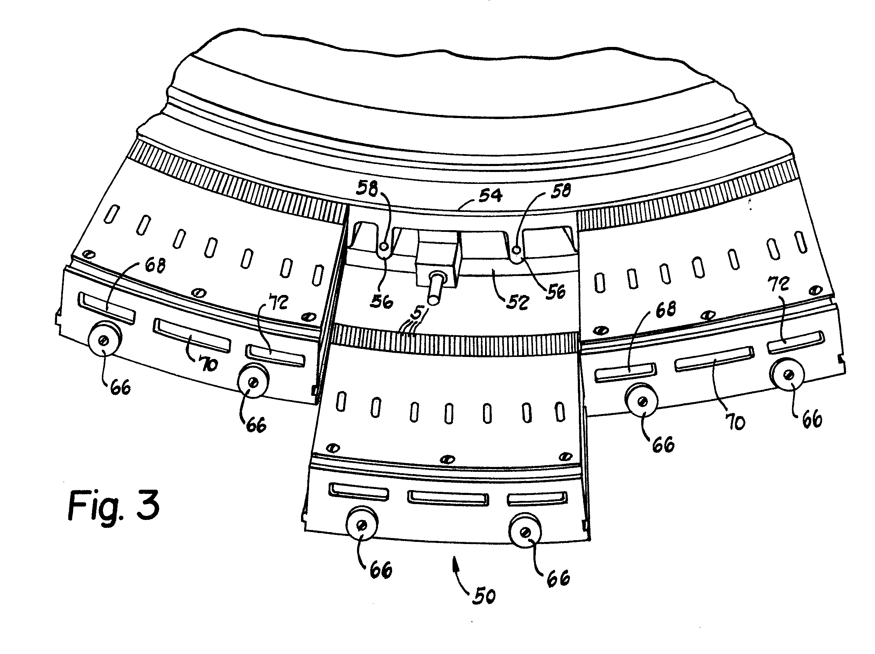

Each of the modules 50 can be easily mounted to or removed from the scanner 12 to facilitate assembly or maintenance of an assembled unit. A stationary gantry 52 supports a number of module mounts 54 (see Figure 3), with each module mount 54 defining a series of fingers 56 which extend radially outward from the module mount 54. These fingers define threaded openings 58 into which threaded connectors 60 on the modules 50 are screwed to mount the modules 50 about the patient aperture 18. During assembly, a first module is screwed into place and others modules 50 comprising the detector array are successively mounted to a module mount 54 in coacting relationship with the first module until an entire array of twenty modules has been positioned in place.

Components of an air conditioning system for directing cool air to the detectors 51 are seen in Figure 2. This air conditioning system comprises a compressor 114, a condensor 116 and two evaporator units 118, 120. Since the detectors in the disclosed fourth generation CT apparatus are all stationary, the cooling system components are also all stationary and in particular are mounted on the four corners of the CT housing between that housing and the detector array 42.

The air conditioning system utilizes a freon coolant to accomplish a heat exchange between cold freon and the relatively warm ambient air surrounding the CT scanner 12. As a first step of the cooling process, the compressor 114 compresses freon in a gaseous state to an elevated temperature and directs this compressed freon to the condensor 116.

At the condensor 116, the compressed freon is allowed to expand and cool. Condensor coils inside the condensor unit 116 heat ambient air as it is forced past the condensor coils and exited from the system by a hot air exhaust 117. As the liquid freon leaves the condensor 116, it passes through a filter 119 which purifies the freon. The output from the condensor 116 leads to a tee 121 which divides the freon into separate paths 123, 125 to the two evaporator units 118, 120. Inside the evaporator units the liquid freon evaporates, and as it does so cools air which is blown from the back of the computed tomography unit through the condensor units 118, 120 to a pair of outlets 127, 129 on each of the evaporator units.

The cool air is forced through each of the modules 50 to reduce the temperature of the detectors to a point below ambient. This reduction in temperature reduces the noise of the detector outputs.

Each detector comprises a scintillation crystal coupled to a photodiode which in turn is mounted to a ceramic mounting block. The detector is encapsulated in an aluminum can which reflects visible light while allowing x-radiation to be freely transmitted to the scintillation crystal. In operation, the x-radiation from the x-ray tube impinges upon the scintillation crystal which converts the x-radiation to visible light which in turn affects the current flow in the photodiode. Changes in current produced by the x-radiation are converted from an analog current signal into a sequence of pulses which are counted.

Electronics for generating these pulses in response to current changes in the photodiode are known in the art. The pulses are then counted and divided by the time period in which they are counted to obtain an indication of the intensity of the x-radiation impinging upon the detector at a given time. Circuitry for performing this counting function is disclosed in U.S. Patent No. 4,052,620 to Brunnett which is assigned to the assignee of the present invention. That prior patent is incorporated herein by reference.

The steps of detecting the radiation 150 and generating the pulses 151 as well as determining the intensity 152 are depicted in a flow chart (Figure 4) schematically describing the computed tomography process. These three steps 150, 151, 152 are followed by a logging of the data and a storing 154 of that data in the computer. The x-ray intensity measured by a detector is inversely proportional to the exponential of the line integral of the density of the subject for a given radiation path. The logarithm of the intensity data yields density data rather than attenuation information.

0109205

The remaining steps in the computed tomography process are performed by the computer 16. The computer first performs a series of calibration and correction calculations 156 on the data. These calculations are based upon data obtained during a CT set-up phase. These calculations take into account variations in detector sensitivity, gain, and offsets in the electronics. Once these calibration steps have been completed, a digital filtering step 158 is performed where all data from each detector is filtered in accordance with one of a number of techniques known in the art. Two known techniques employ convolution or fast fourier transforming (FFT) of data. In the later case the process consists of performing a forward FFT of the data, multiplying the transformed data by a spacial frequency filter 160 and then performing an inverse FFT 162 to produce the filtered data.

At a next stage of the computed tomography process, filtered data for those detectors which are not functioning is assigned 164 based upon the filtered data from those detectors which are supplying valid data. Finally, all data, both from those detectors that are functioning and those which are not, are backprojected 166 into a memory to produce an image of a particular patient slice under examination. Once this backprojection process has been completed, this data is again stored and utilized in imaging 168 a picture of this slice on the console 14.

As the x-ray source 40 rotates about the patient aperture, it successively irradiates each detector comprising the detector array 42. A portion of that array 42 is shown schematically in Figure 5. In accordance with a preferred design, the fan of x-radiation irradiates a portion of the total detectors comprising the array 42. For illustration purposes, however, the Fig-

16          0109205

ure 5 representation shows substantially ·fewer detectors than when are normally irradiated.

If the subject inside the patient aperture 18 were generally uniform in density, that portion of the radiation which misses the subject is essentially unattenuated and the x-radiation passing through the thickest part of the subject will be attenuated to the greatest extent.

Shown in Figure 6 is a plot of the data stored in the computer for a given detector as that detector senses radiation from the source 40 in its orbital path about the cross-section subject shown in Figure 5. As the source 40 rotates about the subject initially, no attenuation in radiation occurs and the corresponding data values for a particular detector will be a minimum. As the x-radiation source continues to move, the radiation striking the detector of interest will begin to be attenuated by the subject and the corresponding data values stored in the computer will begin to increase until the radiation impinging upon that detector undergoes maximum ~~alternatives~~ ATTENUATION at which point the data will peak Ⓐ at a maximum value. As the source 40 continues to move, the attenuation will be less as the radiation passes through a smaller cross-section until the radiation again passes unattenuated from the source 40 to the detector of interest.

The entire data array for a particular detector over the period that detector is being irradiated by the source 40 is known as a view. The views from each of the detectors comprising the detector array 42, are stored on the computer's disc storage system prior to the digital filtering of that data. In accordance with a preferred design, each detector view comprises 1,024 data values, each of which must be digitally filtered, and then backprojected across a grid or mapping of the cross-section of interest. The numbers listed in table
1 comprises a limited number of typical data values
that have been filtered from eight adjacent detectors
comprising the array 42. Since a particular view for a
detector comprises 1,024 of these points, it is appreciated that only a very limited portion of the view is
shown in table 1.

|   | 1 | 2 | 3 | 4 | 5 | 6 | 7 | 8 |
|---|---|---|---|---|---|---|---|---|
| 1 | 103 | 201 | 201 | 203 | 203 | 209 | 200 | 203 |
| 2 | 104 | 101 | 201 | 206 | 204 | 205 | 205 | 203 |
| 3 | 103 | 109 | 106 | 209 | 205 | 202 | 203 | 205 |
| 4 | 104 | 105 | 104 | 106 | 209 | 202 | 208 | 208 |
| 5 | 105 | 104 | 107 | 110 | 105 | 205 | 200 | 202 |
| 6 | 104 | 100 | 104 | 103 | 103 | 102 | 208 | 207 |
| 7 | 105 | 100 | 107 | 101 | 108 | 107 | 103 | 208 |
| 8 | 100 | 103 | 104 | 102 | 107 | 108 | 109 | 109 |

Various techniques are known for determining which
detectors have malfunctioned. One procedure is to take
a calibration scan and view the backprojected results
of that scan. Streaks or artifacts pointing radially
away from a single point at the edge of the picture are
a strong indication that a detector (or its associated
circuitry) has malfunctioned. This suspicion can be
confirmed by testing the detector output directly.
Another technique is to display the data from a sequence
of detectors on the console and look for variations
which should not exist. This is possible, for example,
when the scanner is running a test scan on air or water
and there should be only small variations in the detector
output around the array 42.

Once it is known which detectors have gone "bad"
missing data for those detectors is assigned by the computer. In accordance with a prior art technique, the missing data values for the non-functioning detector was obtained by summing the data points· from the two detectors on either side of the malfunctioning detector and dividing by two. In the illustration, therefore, if the prior art averaging technique were utilized, detector #4 would be assigned values corresponding to the average of numbers directly above and below, thus a value of 155 would be assigned to the 5th data point displayed in table 1. This is to be contrasted with the actual data point of 209 which ideally should be assigned for this data point. Gross miscalcuclations of the data· values for non-functioning detectors caused streaks or artifacts in prior art imaging techniques.

In the discussion that follows-regarding bad detector data approximations the terminology $D(M,N)$ will be used to mean the value for the $N^{TH}$ data point of the Mth detector, in table 1. Thus, $D(4,5)$ has a value of 209 in that table. If the value of $D(4,5)$ is an average of $D(3,5)$ and $D(5,5)$ one obtains 155 for that sample rather than the actual value of 209.

In accordance with the present invention, gradients are· computed in a number of directions about a particular data point to be approximated. The information contained in these gradient values can be used to determine which data points from near-by good detectors should be used to obtain a more accurage estimate of the missing bad detector data point.

To illustrate the procedure, assume that the Mth detector is bad and the adjacent detectors (M-1) and (M+1) are good. The data point $D(M,N)$ can be approximated as follows:

1) define the gradient about point $D(M,N)$ as

$$G(M,N) = \left| D(M-1, N-\omega) - D(M+1, N+\omega) \right|$$

where u is an integer which can be varied between selectable limits of -U to +U.

2) calculate the value of G(M,N) for each value of u.

3) determine the value of u that yielded the smallest value for G(M,N); designate this value of u as $U$ . (A)
min.

4) calculate the value for the bad point D(M,N) as follows

$$D(N,M) = 1/2 \text{ X } [D(M-1, N-U \text{ min}) + D(M+1, N + U\text{min})]$$

To illustrate with a specific example, assume that detector 4 of Table 1 is bad, and that we wish to estimate a value for the 5th data point of detector 4. The following gradient calculations would be made assuming U=2:

$$G(4,5) = |106 - 200| = 94 \qquad \text{for } u= 2$$
$$G(4,5) = |209 - 205| = 4 \qquad \text{for } u= 1$$
$$G(4,5) = |205 - 105| =100 \qquad \text{for } u= 0$$
$$G(4,5) = |202 - 110| = 92 \qquad \text{for } u= -1$$
$$G(4,5) = |203 - 107| = 96 \qquad \text{for } u= -2$$

From the above, it can be seen that u=1 yields the smallest value of G(4,5). Hence

$$\text{Umin} = 1$$

Finally, the estimated value for the bad detector point is

$$D(4,5) = 1/2 \text{ X } (209 + 205) = 207$$

0109205

This approximation is much closer to the ideal value of 209 than 155.

The single bad detector gradient calculation can be extrapolated to a multiple bad detector approximation. Again, assume that the Mth detector is bad and in this case that L is the nearest lower numbered good detector and H is the nearest higher numbered good detector. If:

$$(M-L) \geqslant (H-M), \text{ then}$$

$$G(M,N) = \left| D(L,N-u) - D(H,N + u^1) \right| \text{ where} \qquad \text{(A)}$$

$$u^1 = u \times \frac{(H-M)}{(M-L)}$$

If:

$$(H-M) \geqslant (M-L), \text{ then}$$

$$G(M,N) = \left| D(L,N-u^1) - D(H,N + u) \right| \text{ where}$$

$$u^1 = u \times \frac{(M-L)}{(H-M)} \qquad \text{(A)}$$

If:

$$(H-M) = (M-L), \text{ then } u^1 = u \text{ and}$$

$$G(M,N) = \left| D(L,N-u) - D(H,N+u) \right|$$

If $u^1$ is not an integer, then interpolation (e.g. linear) is used to determine the value of the data point which uses $u^1$.

$G(M,N)$ is evaluated for each integer value of u where $-U \leqslant u \leqslant U$.

The value of u which yields the smallest value of $G(M,N)$ is designated Umin.

The bad detector data is generated as follows:

$$(\text{case I}) \quad (M-L) \geqslant (H-M)$$

$$D(M,N) = 1/2 \left[ D(L,N-U^1 min) + D(H,N + U^1 min) \right] \qquad \text{(A)}$$

21    0109205

where $U^1min = Umin \times \dfrac{(H-M)}{(M-L)}$

(case II)   $(H-M) \geqslant (M-L)$

$D(M,N) = 1/2 [D(L,N-U^1min) + D(H,N + U^1min)]$

where $U^1min = Umin \times \dfrac{(M-L)}{(H-M)}$

To illustrate with a specific example, assume that detectors 3 and 4 are bad.  In generating point $D(4,5)$ it is noted that:

L = 2 and H = 5 so that M − L =
4 − 2 = 2 and H − M = 5 − 4 = 1

so, $u^1 = u \times 1/2$.

Setting U = 2.

$G(4,5) = |201 - 205| = 4$ for u = 2
$G(4,5) = |206 - 155| = 51$ for u = 1
$G(4,5) = |204 - 105| = 91$ for u = 0
$G(4,5) = |205 - 107.5| = 97.5$ for u = −1
$G(4,5) = |205 - 110| = 95$ for u = −2

The smallest value of $G(4,5)$ is for u = 2 so that estimated value of $D(4,5) = 1/2 [201 + 205] = 203$.

A technique somewhat different from the gradient technique is designated the correlation technique.  For a single bad detector let $C(M,N)$ BE A CORRELATION CONSTANT.

$$C(M,N) = \sum_{k=-K}^{K} D(M-1, M-u + k) \times D(M+1, M+u+k)$$

where:

u is an integer that varies from $-U \leq u \leq U$

k is an integer that varies from $-K \leq k \leq K$

The approximation procedure is as follows:

1)   calculate $C(M,N)$ for each value of $u$;

2)   determine which value of $u$ yields the maximum value of $C(M,N)$ and designate that value of $u$ as Umax;

3)   generate bad detector data as follows:

$$D(M,N) = 1/2 \ [D(M-1, \ N-Umax) + D(M+1, \ N+Umax)]$$

Multiple bad detector correlation is also possible

(case I)   $(M-L) \geq (H-M)$

$$C(M,N) = \sum_{k=-k}^{K} D(L,N - u + k) \times D(H,N + u^1 + k)$$

where $u^1 = u \ \dfrac{(H-M)}{(M-L)}$

If $u^1$ is non-integer, interpolation can be used to determine the value of point $(N + u^1 + k)$

Umax = value of $u$ where $C(M,N)$ is maximum

$$U^1max = Umax \ \dfrac{(H-M)}{(M-L)}$$

Bad detector data is determined as follows:

$$D(M,N) = 1/2 \ [D(L,N-Umax) + D(H,N + U^1max)]$$

(Case II)   $(H-M) > (M-L)$

$$C(M,N) = \sum_{k=-k}^{K} D(L,N -u^1 + k) \times D(H,N + u +k)$$

$$D(M,N) = 1/2 \ [D(L,N - U^1max) + D(H,N + U \ max)]$$

To illustrate the correlation technique consider an example of single bad detector. Assume detector 4 has malfunctioned. To determine point 5, i.e. D(4,5) set U = 2 and K = 1.

$$C(4,5) = (109 \times 205) + (106 \times 200) + (209 \times 202) = 83945 \quad \text{for } u=2$$
$$= (106 \times 105) + (209 \times 205) + (205 \times 200) = 94975 \quad \text{for } u=1$$
$$= (209 \times 110) + (205 \times 105) + (202 \times 205) = 85925 \quad \text{for } u=0$$
$$= (205 \times 107) + (202 \times 110) + (203 \times 105) = 65470 \quad \text{for } u=-1$$
$$= (202 \times 104) + (203 \times 107) + (205 \times 110) = 65279 \quad \text{for } u=-2$$

choosing the maximum instead of minimum value of the correlation factor one has $U_{max} = 1$, so that substituting this value in the formula for D(M,N) one has:

$$D(4,5) = 1/2 \ (209 + 205) = 207.$$

To illustrate the correlation technique for multiple bad detectors assume detectors 3, 4, and 5 have gone bad and one wishes to evaluate the point D(4,5).

Let U = 2 and K = 1 and calculate the correlation factors as follows:

$$C(4,5) = (102 \times 102) + (201 \times 208) + (206 \times 207) = 94752 \quad \text{for } u=2$$
$$= (201 \times 103) + (206 \times 102) + (204 \times 208) = 84147 \quad \text{for } u=1$$
$$= (206 \times 103) + (204 \times 103) + (205 \times 102) = 63140 \quad \text{for } u=0$$
$$= (204 \times 104) + (205 \times 103) + (205 \times 103) = 63446 \quad \text{for } u=-1$$
$$= (205 \times 100) + (205 \times 104) + (203 \times 103) = 62729 \quad \text{for } u=-2$$

One sees that $U_{max} = 2$ so that substituting appropriate value in the formula for D gives:

$$D(4,5) = 1/2 \ [201 + 208] = 204.5$$

From the above it is apparent that while the present invention has been described with a degree of particularity, certain modifications in the bad detector approxi-

24

0109205

mation technique could be readily apparent to one skilled in the art. It is the intent therefore, that all such modifications and/or alterations falling within the spirit or scope of the appended claims be protected.

0109205

<u>CLAIMS</u>

1.    Computerized tomography scanning apparatus for imaging a cross-sectional slice of a subject comprising:

an array of closely spaced radiation sensing detectors;

a radiation source rotatably mounted to direct radiation through siad subject to said detector array from a number of positions;

means for storing indications of the radiation intensity after the radiation has been attenuated by said subject as the radiation from said moving source reaches the detector array;

means for approximating intensity values for nonfunctioning detectors in said array by assigning values to said nonfunctioning detectors;  and

means for manipulating the intensity data from those functioning detectors and the values assigned to nonfunctioning detectors to create a reconstructed computed tomography image and for presenting said image in a form for discerning density variations in the cross-sectional slice;  characterised in that, in said means for approximating intensity values for non-functioning detectors, said assigned values correspond to a preferred function of the intensity values from closely positioned functioning detectors.

2.    A method in computerized tomography employing a stationary detector array for imaging the cross-section of a patient comprising the steps of:

ascertaining which of the multiple detectors comprising the detector array are functioning and which are not;

directing a spread beam of radiation from a moving radiation source through the cross-section to impinge upon successive portions of said stationary array;

sensing radiation intensity from those detectors which are functioning;

assigning computed tomography data for the non-functioning detectors;   and

using the data from those detectors which are functioning and the data assigned to those detectors which are not functioning to reconstruct an image of said cross-section suitable for determining density variations in said cross-section;   characterised in that the step of assigning computed tomography data for the nonfunctioning detectors is performed by calculating a preferred difference in data between closely adjacent operating detectors and then calculating and assigning data to each non-functioning detector based upon the preferred difference.

3.     A method according to Claim 2 wherein the calculating step comprises finding a minimal difference between radiation intensities from closely adjacent functioning detectors at both the same and at different times and using this difference to provide intensity readings which are then convolved and backprojected.

4.     A method according to Claim 2 or Claim 3 wherein the calculating step is performed after intensity data from those functioning detectors has been convolved so that the preferred difference is calculated on convolved data.

5.     A method for imaging a cross-sectional slice of a subject in computed tomography comprising the steps of:

positioning a plurality of closely spaced radiation detectors in a circular array;

positioning the subject so that said slice coincides with a center of said circular array;

orbiting a source of x-radiation around said subject along a circular path having a radius less than the  radius of said circular detector array;

sensing radiation intensities along different x-radiation paths as the radiation is attenuated by said subject;

storing the sensed intensities from those detectors in said array which are functioning;

filtering the intensity data from said functioning detectors to provide filtered data;

assigning filtered data values to nonfunctioning detectors; and

using the data from both functioning and non-functioning detectors to backproject an image of said slice; characterised by the steps of calculating a difference in filtered data between closely spaced functioning detectors at closely spaced times and choosing a preferred difference to define a gradient; and

assigning filtered data values to non-functioning detectors on said gradient by applying a function of data from functioning detectors along said gradient.

6. A method according to Claim 5 wherein the assigning step is accomplished by interpolating the data between closely spaced functioning detectors.

7. A method in computerized tomography employing a stationary detector array for imaging the cross-section of a patient comprising the steps of:

ascertaining which of the multiple detectors comprising the detector array are functioning and which are not;

directing a spread beam of radiation from a moving radiation source through the cross-section to impinge upon successive portions of said stationary array;

sensing radiation intensity from those detectors which are functioning;

assigning computed tomography data for the non-functioning detectors; and

using the data from those detectors which are functioning and the data assigned to those detectors which are not functioning to reconstruct an image of said cross-section suitable for determining density variations in said cross-section; characterised in that the step of assigning computed tomography data for the nonfunctioning detectors is performed by calculating a preferred correlation in data between closely adjacent operating detectors and then calculating and assigning data to each non-functioning detector based upon the preferred correlation.

Fig.1

Fig. 2

Fig. 3

0109205

DETECT RADIATION /150 → GENERATE PULSES /151 → CALCULATE INTENSITY /152 → STORE DATA /154

CALIBRATE & CORRECT 156 → DIGITAL FILTERING 158 → SUPPLY BAD DETECTOR DATA 164 → BACK PROJECT 166

MULTIPLY 160

INVERSE TRANSFORM 162

IMAGE CROSS SECTION 168

Fig. 4

Fig. 5

Fig. 6

Fig. 7